# EUROPEAN PATENT SPECIFICATION

(11) **EP 4 480 439 B1**
(45) Date of publication and mention of the grant of the patent: **03.06.2026**
(21) Application number: 24164054.9
(22) Date of filing: 18.03.2024
(51) Int. Cl.: A61B 34/20

(54) **APPARATUS TO OVERLAY INFORMATION ON ENDOSCOPIC IMAGES**
VORRICHTUNG ZUR ÜBERLAGERUNG VON INFORMATIONEN AUF ENDOSKOPISCHEN BILDERN
APPAREIL POUR SUPERPOSER DES INFORMATIONS SUR DES IMAGES ENDOSCOPIQUES

(30) Priority: 18.04.2023 US 202363460050 P; 08.02.2024 US 202418436596
(43) Date of publication of application: 25.12.2024
(73) Proprietor: Acclarent, Inc., Irvine, CA 92618 (US); Biosense Webster (Israel) Ltd., 2066717 Yokneam (IL)
(72) Inventor: HOD, Uriel, 2066717 Yokneam (IL); BUSTAN, Itamar, 2066717 Yokneam (IL); WRIGHT, Alison D., Irvine, 92618 (US); AUERBACH, Shmuel, 2066717 Yokneam (IL); PALUSHI, Jetmir, 92620 Irvine (IL)
(74) Representative: Carpmaels & Ransford LLP

(56) References cited:
- US-A1- 2014 336 669
- US-A1- 2016 008 083
- US-A1- 2019 192 232
- US-A1- 2020 331 147

## Description

### BACKGROUND

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. An example of an electromagnetic IGS navigation system that may be used in IGS procedures is the CARTO^{®} 3 System by Biosense-Webster, Inc., of Irvine, California. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit electromagnetic fields and/or are responsive to externally generated electromagnetic fields) are used to perform the procedure while the sensors send data to the computer indicating the current position of each medical procedure instrument. The computer correlates the data it receives from the sensors with the digital map that was created from the preoperative tomographic scan. The tomographic scan images are displayed on a video monitor along with an indicator (e.g., crosshairs or an illuminated dot, etc.) showing the real-time position of each medical procedure instrument relative to the anatomical structures shown in the scan images. The surgeon is thus able to know the precise position of each sensor-equipped instrument by viewing the video monitor even if the surgeon is unable to directly visualize the instrument itself at its current location within the body.

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guide wire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus.

It may also be desirable to ablate tissue within the ear, nose, or throat of a patient. For instance, such ablation may be desirable to remodel tissue (e.g., to reduce the size of a turbinate), to provide denervation (e.g., to disable the posterior nasal nerve), and/or for other purposes. To achieve ablation, an end effector with one or more needle electrodes or other kind(s) of tissue contacting electrodes may be activated with monopolar or bipolar RF energy. Such ablation procedures may be carried out in conjunction with a dilation procedure or separately from a dilation procedure.

It may also be desirable to provide easily controlled placement of a dilation catheter, ablation instrument, or other ENT instrument in an anatomical passageway, including in procedures that will be performed only by a single operator. While several systems and methods have been made and used to position an ENT instrument in an anatomical passageway, it is believed that no one prior to the inventors has made or used the invention described in the appended claims.

US 2019/192232 A1 describes use of augmented reality to assist navigation during medical procedures. A disclosed augmented reality navigation system provides tool location visibility for invasive procedures through the use of location sensors included on a camera and/or on the tools used during a procedure. A location tracking system determines and monitors the locations of the tools and camera based on the characteristics of signals detected by the sensors (such as amplitude or frequency of electromagnetic sensors). A registration process correlates a 3D model of the patient (generated, e.g., via a medical scan such as a computed tomography ("CT") scan) to the actual location of that patient. Thus the location of tools and the camera is tracked relative to the location of the anatomy of the patient. This relative location tracking allows for information related to the procedure to be composited with the image generated by the camera. For example, because the position of the camera relative to the tools is known, the location of the tools can be displayed within the image even when the tools are not visible to a human operator due to being occluded by tissue of the patient. This displaying is accomplished through standard three-dimensional ("3D") rendering techniques that render an image of an object relative to the position of a camera. Additional information, such as a visualization of tools, rendering of anatomy, including display of three-dimensional structures of anatomy such as an artery or a network of blood vessels, a network of nerves, or the pre-operation identified borders of a tumor that is intended to be removed, or other information, could additionally or alternatively be shown in such a composited image.

US 2020/331147 A1 describes a method for indicating a tool position or orientation relative to images being displayed on a computer display screen when the tool is occluded within a viewing area of the screen. Figure 7 of US 2020/331147 A1 illustrates, as an example, a GUI generated screen that is displayed on a monitor, wherein a tool is positioned so as to be within a viewing area, but the end effector of the tool is occluded by an object. Although the end effector of the tool is occluded by the object, a ghost image (e.g., a computer model) of the end effector is shown at the proper position and orientation over the object.

US 2016/008083 A1 describes an exemplary sinuplasty procedure in which a guidewire is inserted into a nasal sinus. Circuitry coupled to a sensing coil acquires signals from the sensing coil, while field generators are transmitting their magnetic fields. A processor applies a calibration relationship to the signals, and together with a registration finds the location and orientation of the sensing coil. An indication of the location and orientation of the sensing coil, i.e., of the distal end of the guidewire, may be overlaid onto a registered, preexisting image of the sinuses of the patient. The composite image, of the sinuses of the patient and the distal end of the guidewire, may be displayed to a physician on a screen.

US 2014/336669 A1 describes haptic gloves and surgical robot systems. The haptic glove is a glove that can be wearable on hands of the operator. As the operator moves the hands while wearing the haptic glove, a depth sensor recognizes the shape, position, posture, gesture, and motion of the haptic glove, to display an image displayed on a display combined with a representation of the haptic glove in an overlay manner. The display of the master system may display an actual image of the inside of the body of a patient collected through an endoscope and a three-dimensional image of a medical image obtained before the patient has a surgery.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of an example of a surgery navigation system being used on a patient seated in an example of a medical procedure chair;
FIG. 2A depicts a front perspective view of an example of an instrument with a slider in a proximal position, such that a working element shaft of the instrument is retracted proximally relative to an open distal end of a shaft assembly of the instrument;
FIG. 2B depicts a front perspective view of the instrument of FIG. 2A, with the slider in a distal position, such that the working element shaft is extended distally relative to the open distal end of the shaft assembly;
FIG. 3 depicts a perspective view of a distal portion of the instrument of FIG. 2A;
FIG. 4 depicts a graphical user interface (GUI) view of an image captured by an endoscope;
FIG. 5 depicts a GUI view of an arrangement of preoperative images;
FIG. 6 depicts a flow diagram of a process for overlaying supplemental information on a medical procedure navigation display;
FIG. 7 depicts a GUI view of an arrangement of preoperative and operative images;
FIG. 8 depicts a flow diagram of a process for overlaying supplemental information on a medical procedure navigation display;
FIG. 9 depicts a GUI view of an image captured by an endoscope with a semi-transparent overlay; and
FIG. 10 depicts a GUI view of an image captured by an endoscope with a non-transparent overlay.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive. Embodiments of the invention are described by section IV. Features of the invention, and additional optional features, are described in greater detail by other sections.

For clarity of disclosure, the terms "proximal" and "distal" are defined herein relative to a surgeon, or other operator, grasping a medical procedure instrument having a distal medical procedure end effector. The term "proximal" refers to the position of an element arranged closer to the surgeon, and the term "distal" refers to the position of an element arranged closer to the medical procedure end effector of the medical procedure instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that medical procedure instruments such as those disclosed herein may be used in a variety of orientations and positions not limited to those shown and described herein.

As used herein, the terms "about" and "approximately" for any numerical values or ranges indicate a suitable dimensional tolerance that allows the part or collection of components to function for its intended purpose as described herein.

### I. Example of an Image Guided Surgery Navigation System

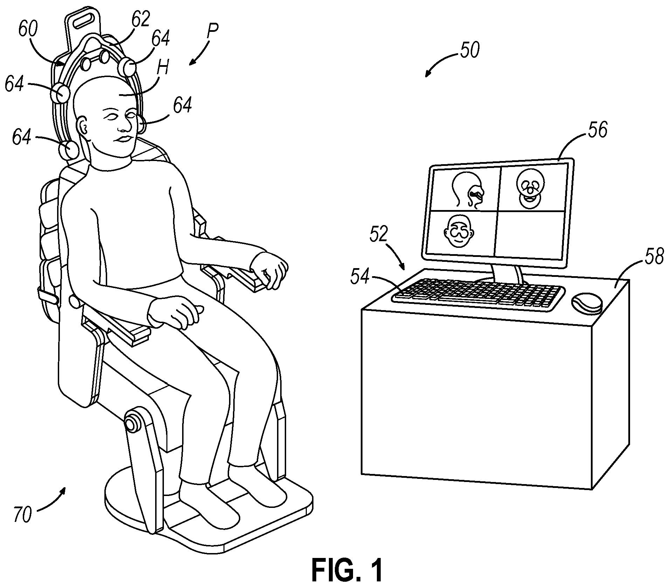

When performing a medical procedure within a head (H) of a patient (P), it may be desirable to have information regarding the position of an instrument within the head (H) of the patient (P), particularly when the instrument is in a location where it is difficult or impossible to obtain an endoscopic view of a working element of the instrument within the head (H) of the patient (P). FIG. 1 shows an example of a IGS navigation system (50) enabling an ENT procedure to be performed using image guidance. In addition to or in lieu of having the components and operability described herein IGS navigation system (50) may be constructed and operable in accordance with at least some of the teachings of U.S. Pat. No. 7,720,521, entitled "Methods and Devices for Performing Procedures within the Ear, Nose, Throat and Paranasal Sinuses," issued May 18, 2010; and U.S. Pat. Pub. No. 2014/0364725, entitled "Systems and Methods for Performing Image Guided Procedures within the Ear, Nose, Throat and Paranasal Sinuses," published December 11, 2014, now abandoned.

IGS navigation system (50) of the present example comprises a field generator assembly (60), which comprises set of magnetic field generators (64) that are integrated into a horseshoe-shaped frame (62). Field generators (64) are operable to generate alternating magnetic fields of different frequencies around the head (H) of the patient (P). An instrument, such as any of the instruments described below, may be inserted into the head (H) of the patient (P). Such an instrument may be a standalone device or may be positioned on an end effector. In the present example, frame (62) is mounted to a chair (70), with the patient (P) being seated in the chair (70) such that frame (62) is located adjacent to the head (H) of the patient (P). By way of example only, chair (70) and/or field generator assembly (60) may be configured and operable in accordance with at least some of the teachings of U.S. Pat. No. 10,561,370, entitled "Apparatus to Secure Field Generating Device to Chair," Issued February 18, 2020.

IGS navigation system (50) of the present example further comprises a processor (52), which controls field generators (64) and other elements of IGS navigation system (50). For instance, processor (52) is operable to drive field generators (64) to generate alternating electromagnetic fields; and process signals from the instrument to determine the location of a navigation sensor in the instrument within the head (H) of the patient (P). Processor (52) comprises a processing unit (e.g., a set of electronic circuits arranged to evaluate and execute software instructions using combinational logic circuitry or other similar circuitry) communicating with one or more memories. Processor (52) of the present example is mounted in a console (58), which comprises operating controls (54) that include a keypad and/or a pointing device such as a mouse or trackball. A physician uses operating controls (54) to interact with processor (52) while performing the medical procedure.

While not shown in FIG. 1, the instrument may include a navigation sensor that is responsive to positioning within the alternating magnetic fields generated by field generators (64). A coupling unit (not shown) may be secured to the proximal end of the instrument and may be configured to provide wired or wireless communication of data and other signals between console (58) and the instrument. In some versions, the navigation sensor of the instrument may comprise at least one coil at or near the distal end of the instrument. When such a coil is positioned within an alternating electromagnetic field generated by field generators (64), the alternating magnetic field may generate electrical current in the coil, and this electrical current may be communicated along the electrical conduit(s) in the instrument and further to processor (52) via the coupling unit. These signals may enable IGS navigation system (50) to determine the location of the distal end of the instrument within a three-dimensional space (i.e., within the head (H) of the patient (P), etc.). To accomplish this, processor (52) executes an algorithm to calculate location coordinates of the distal end of the instrument from the position related signals of the coil(s) in the instrument.

Processor (52) uses software stored in a memory of processor (52) to calibrate and operate IGS navigation system (50). Such operation includes driving field generators (64), processing data from the instrument, processing data from operating controls (54), and driving display screen (56). Processor (52) is further operable to provide video in real time via display screen (56), showing the position of the distal end of the instrument in relation to a video camera image of the patient's head (H), a CT scan image of the patient's head (H), and/or a computer-generated three-dimensional model of the anatomy within and adjacent to the patient's nasal cavity. Display screen (56) may display such images simultaneously and/or superimposed on each other during the medical procedure. Such displayed images may also include graphical representations of instruments that are inserted in the patient's head (H), such that the operator may view the virtual rendering of the instrument at its actual location in real time. By way of example only, display screen (56) may provide images in accordance with at least some of the teachings of U.S. Pat. No. 10,463,242, entitled "Guidewire Navigation for Sinuplasty," issued November 5, 2019. In the event that the operator is also using an endoscope, the endoscopic image may also be provided on display screen (56).

The images provided through display screen (56) may help guide the operator in maneuvering and otherwise manipulating instruments within the patient's head (H). It should also be understood that other components of a medical procedure instrument and other kinds of medical procedure instruments, as described below, may incorporate a navigation sensor like the navigation sensor described above.

### II. Example of an ENT Instrument with Steerable Guide and Working Element

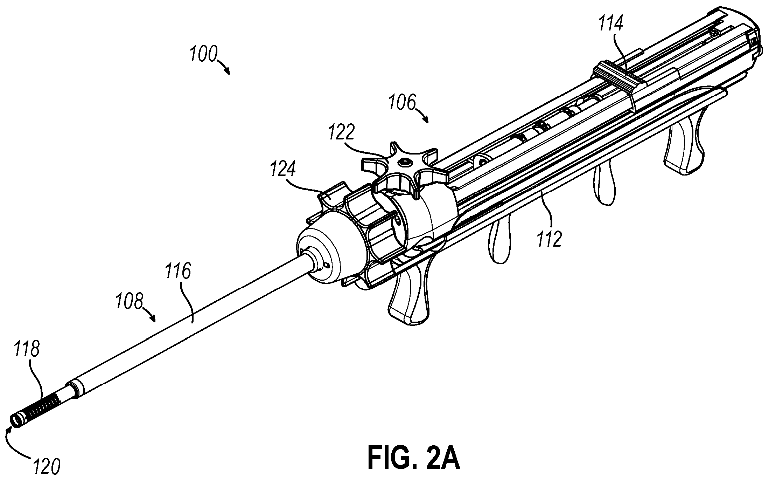

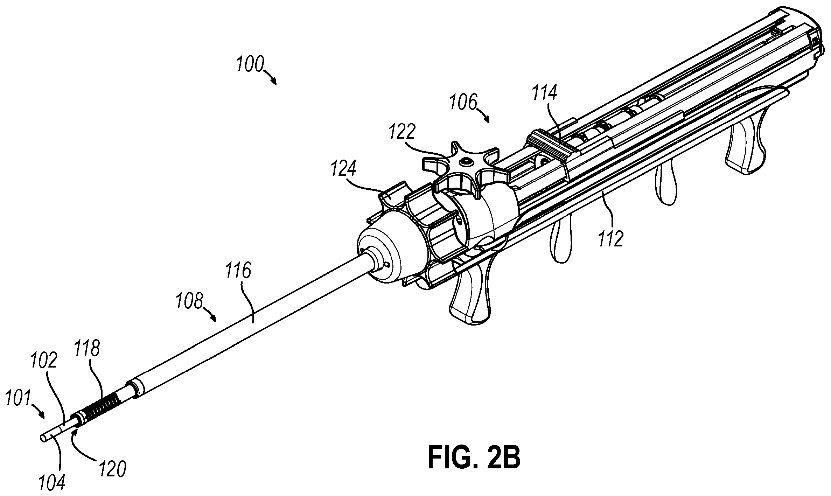

FIGS. 2A-2B show an example of an instrument (100) that may be used to guide a working element (101) (FIG. 2B) into an anatomical passageway. As shown in FIG. 2B, working element (101) includes a shaft (102) and an end effector (104). In some versions, working element (101) may include a dilation catheter. In this regard, end effector (104) may have one or more balloons or other dilators, such that instrument (100) may be used to guide end effector (104) of working element (101) into an anatomical passageway (e.g., an ostium of a paranasal sinus, a larynx, a Eustachian tube, etc.) to thereby dilate the anatomical passageway. In addition, or alternatively, working element (101) may include an RF energy delivery catheter with one or more RF electrodes, such that instrument (100) may be used to guide end effector (104) of working element (101) into an anatomical passageway to deliver RF energy to tissue in or near the anatomical passageway. Alternatively, working element (101) may include any other suitable type of ENT treatment device. Moreover, working element (101) may include one or more navigation sensors that cooperate with IGS navigation system (50) as described above, to provide signals indicating the real-time position of end effector (104).

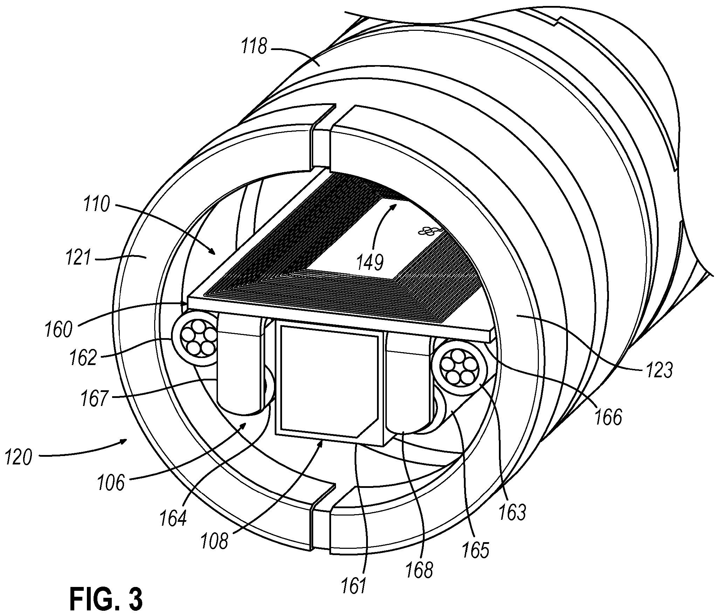

Instrument (100) of this example includes a handle assembly (106) and a shaft assembly (108). In some versions, handle assembly (106) and/or shaft assembly (108) includes one or more navigation sensors (e.g., navigation sensor assembly (110) as described below) that cooperate with IGS navigation system (50) as described above, to provide signals indicating the real-time position of corresponding portions of instrument (100). Instrument (100) may be coupled with an inflation fluid source (not shown), which may be operable to selectively supply an inflation fluid to a balloon (not shown) of end effector (104). In addition, or alternatively, instrument (100) may be coupled with an RF generator (not shown), which may be operable to generate RF electromedical procedure energy for delivery to tissue via electrodes (not shown) of end effector (104) and/or via electrodes (121, 123) (FIG. 3) at distal end (120) shaft assembly (108) to thereby ablate, electroporate, or apply resistive heating to the tissue. Instrument (100) may also be coupled with IGS navigation system (50) to communicate position-indicative signals from navigation sensors of working element (101), handle assembly (106), and/or shaft assembly (108) (e.g., navigation sensor assembly (110) (FIG. 3)) to IGS navigation system (50).

Handle assembly (106) of this example includes a body (112) and a slider (114). Slider (114) is operable to translate longitudinally relative to body (112). Slider (114) is coupled with working element (101) and is thus operable to translate working device (101) longitudinally between a proximally retracted position (FIG. 2A) and a distally extended position (FIG. 2B).

Shaft assembly (108) of the present example includes a rigid portion (116), a flexible portion (118) distal to rigid portion (116), and an open distal end (120). A pull-wire (not shown) is coupled with flexible portion (118) and with a deflection control knob (122) of handle assembly (106). Deflection control knob (122) is rotatable relative to body (112) to selectively retract the pull-wire proximally. As the pull-wire is retracted proximally, flexible portion (118) bends and thereby deflects distal end (120) laterally away from the longitudinal axis of rigid portion (116). Deflection control knob (122), the pull-wire, and flexible portion (118) thus cooperate to impart steerability to shaft assembly (108). By way of example only, such steerability of shaft assembly (108) may be provided in accordance with at least some of the teachings of U.S. Pat. Pub. No. 2021/0361912, entitled "Shaft Deflection Control Assembly for ENT Guide Instrument," published November 25, 2021; and/or U.S. Pat. No. 11,376,401, entitled "Deflectable Guide for Medical Instrument," issued June 15, 2022, in its entirety. Other versions may provide some other kind of user input feature to drive steering of flexible portion (118), instead of deflection control knob (122). In some alternative versions, deflection control knob (122) is omitted, and flexible portion (118) is malleable. In still other versions, the entire length of shaft assembly (108) is rigid.

Shaft assembly (108) is also rotatable relative to handle assembly (106), about the longitudinal axis of rigid portion (116). Such rotation may be driven via rotation control knob (124), which is rotatably coupled with body (112) of handle assembly (106). Alternatively, shaft assembly (108) may be rotated via some other form of user input; or may be non-rotatable relative to handle assembly (106).

A working lumen extends longitudinally from an open proximal end of shaft assembly (108) all the way to open distal end (120) and is configured to slidably receive working element (101), such that shaft assembly (108) may receive working element (101) at the open proximal end, and such that shaft assembly (108) may guide working element (101) out through open distal end (120).

As shown in FIG. 3, the distal region of shaft assembly (108) further includes navigation sensor assembly (110). Navigation sensor assembly (110) includes one or more coils (e.g., a single-axis sensor, a dual-axis sensor, a three-axis sensor, six-axis sensor, etc.) that are responsive to positioning within the alternating magnetic fields generated by field generators (64). In some versions, these one or more coils are formed as traces in a flex circuit. When navigation sensor assembly (110) is positioned within an alternating electromagnetic field generated by field generators (64), the alternating magnetic field may generate electrical current in the coils, and this electrical current may be communicated along electrical conduit(s) in instrument (100) and further to processor (52) via a coupling unit. These signals may enable IGS navigation system (50) to determine the location of distal end (120) within a three-dimensional space (i.e., within the head (H) of the patient (P), etc.). To accomplish this, processor (52) executes an algorithm to calculate location coordinates of the distal end (120) from the position-indicative signals generated by navigation sensor assembly (110) in response to the alternating magnetic fields generated by field generators (64).

As also shown in FIG. 3, the distal region of shaft assembly (108) further includes a visualization and irrigation assembly (108), which is disposed within shaft assembly (108) directly below navigation sensor assembly (110). Visualization and irrigation assembly (108) is operable to provide visualization and irrigation at a target tissue site distal to distal end (120) of shaft assembly (108). Visualization and irrigation assembly (108) of this example includes a plate member (160), a camera (161), a pair of illuminating elements (162, 163), and a pair of fluid conduits (164, 165). Camera (161) may be in the form of a camera that is suitably sized to fit within shaft assembly (108) while still permitting space for working channel (149) to extend along shaft assembly (108), thereby permitting additional instrumentation, suction, fluids, etc., and/or working element (104), to pass through open distal end (120) adjacent to camera (161). It should be understood that versions of instrument (100) including camera (161) may serve as a type of endoscope.

Illuminating elements (162, 163) are configured and operable to illuminate the field of view of camera (161). Conduits (164, 165) laterally flank camera (161) in this example. One or both of conduits (164, 165) may be in fluid communication with a source of liquid (e.g., saline, etc.) and/or a source of suction. In versions where at least one of conduits (164, 165) is in communication with a source of liquid, such conduit(s) (164, 165) may be used to deliver such liquid to a distal end of camera (161). By flushing the distal end of camera (161) with liquid, conduits (164, 165) may be used to keep the distal end of camera (161) clear of debris and thereby maintain appropriate visualization via camera (161).

Plate member (160) of this example includes a plate (166) and a pair of transversely extending tabs (167, 168). Plate (166) is positioned over camera (161) and may thus serve to shield camera (161) from getting snagged and perhaps damaged by working element (104) and/or other instruments that are advanced along working channel (149). Tabs (167, 168) are positioned to correspond with the locations of respective distal ends of conduits (164, 165). Tab (167) may be further positioned to leave a gap (not shown) between the proximal face of tab (167) and the distal end of conduit (164), and a similar gap may be left between the proximal face of tab (168) and the distal end of conduit (165). These gaps may be sized to allow liquid to escape from the distal ends of conduits (164, 165); and to allow suction to be applied via the distal ends of conduits (164, 165). However, the presence of tabs (167, 168) may assist in diverting liquid expelled via the distal ends of conduits (164, 165) toward the distal end of camera (161) and thereby assist in flushing debris away from camera (161).

### III. Example of Overlaying Instrument Data onto Images

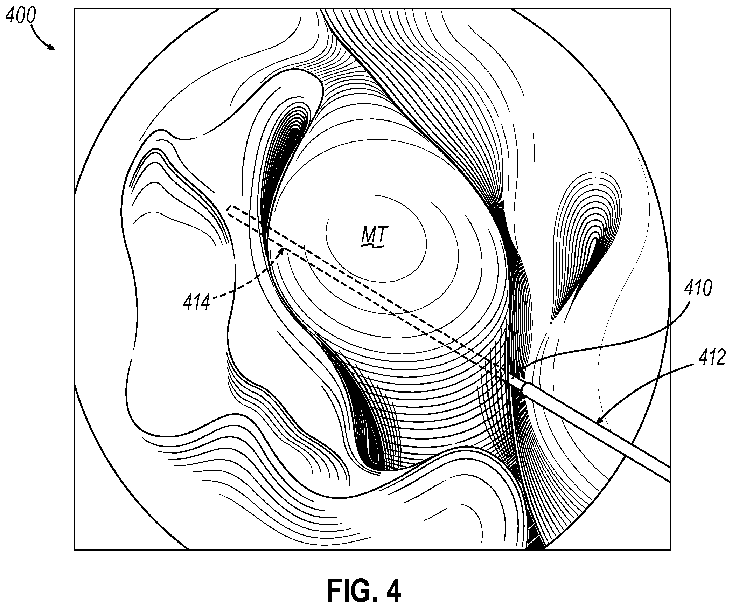

FIG. 4 shows an example image (400), captured by camera (161) within a patient (P), where an instrument (410) is inserted into the patient within the field of view of camera (161). By way of example only, instrument (410) may include a dilation catheter, an ablation instrument, a curette, a microdebrider or other shaver, a probe, an irrigation instrument, a suction instrument, and/or any other suitable kind of instrument. In some variations, image (400) may include a portion of working element (101) protruding from distal end (120) of shaft assembly (108), in addition to or in lieu of instrument (410). Instrument (410) may thus be omitted in some scenarios. While image (400) is captured by camera (161) in this example, the teachings herein may be readily applied to scenarios where image (400) is captured by some other kind of endoscope or imaging device. It should therefore be understood that, while the teachings herein may be applied in contexts where instrument (100) is used, the teachings herein may also be applied in contexts where instrument (100) is not used.

While image (400) clearly shows the anatomical structures and instrument (410) within the field of view of camera (161), it may be desirable provide additional information in images (400) captured via camera (161). For instance, such additional information may include the kind of working element (101) that is disposed in instrument (100); the kind of other instrument (410) that is inserted in the patient with instrument (100); structural characteristics of working element (101) or instrument (410), such as length, bend angle, diameter, etc.; operational characteristics of working element (101) or instrument (410), such as depth of insertion, activation status, etc.; and/or other information. With the additional information being provided in the form of one or more overlays on an image (400) from camera (161), the operator may readily observe the additional information while observing the image (400) from camera (161) during a procedure, without the operator needing to look away from image (400) to observe the additional information.

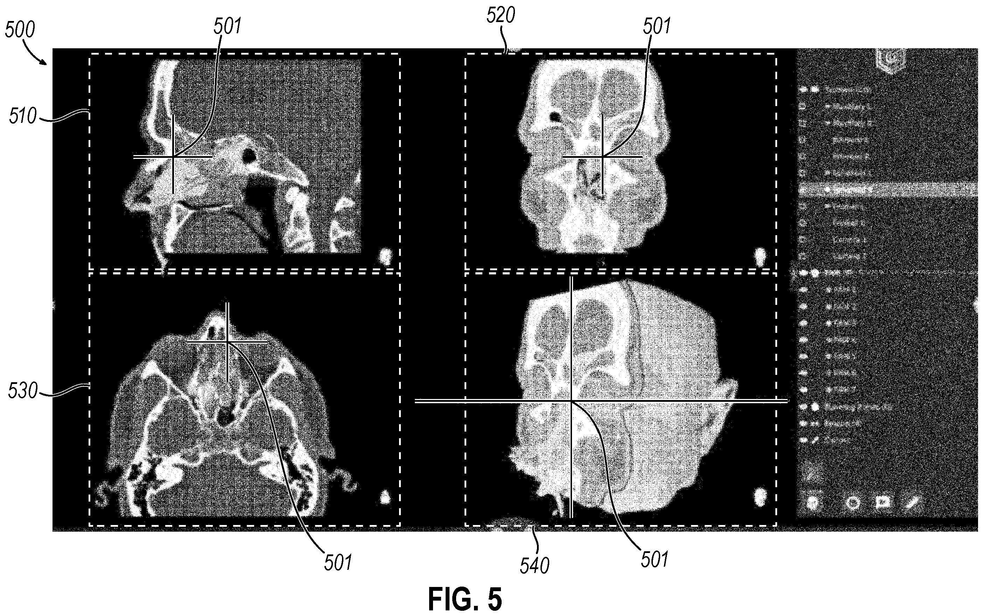

FIG. 5 shows an example of a user interface (500) that may be provided via display screen (56) during use of navigation system (50). In this example, user interface includes an array of preoperative images (510, 520, 530, 540). Preoperative images (510, 520, 530, 540) may include, or be based on, various kinds of preoperative images of the patient (P), such as CT scans, MRI scans, etc. Preoperative image (510) includes a two-dimensional cross-sectional image of the head (H) of the patient (P) taken along a sagittal plane. Preoperative image (520) includes a two-dimensional cross-sectional image of the head (H) of the patient (P) taken along a coronal plane. Preoperative image (530) includes a two-dimensional cross-sectional image of the head (H) of the patient (P) taken along a transverse plane. Preoperative image (530) includes a three-dimensional image of the head (H) of the patient (P), with a cross-section taken along a coronal plane. User interface (500) also includes a crosshairs (501) within each preoperative image (510, 520, 530, 540). Each crosshairs (501) indicates the real-time position of distal end (120) shaft assembly (108). Processor (52) determines where to locate crosshairs (501) within each preoperative image (510, 520, 530, 540) based on position-indicative signals from navigation sensor assembly (110). As the operator moves distal end (120) within the patient (P), the location of crosshairs (501) moves on user interface (500) accordingly. In addition, the cross-sectional planes of preoperative images (510, 520, 530, 540) may also move in user interface (500) as distal end (120) moves within the patient (P).

As noted above, it may be beneficial to include additional information about working element (101) or instrument (410) in an image (400) captured by camera (161), to enable the operator to observe the additional information without needing to look away from image (400). It may also be beneficial to include such additional information within user interface (500) (e.g., as an overlay), to enable the operator to observe the additional information without needing to look away from user interface (500). In addition, it may be beneficial to integrate image (400) into user interface (500). Thus, some versions of display screen (56) may render a combination of image (400), preoperative images (510, 520, 530, 540), and one or more overlays, etc., providing additional information about working element (101) or instrument (410).

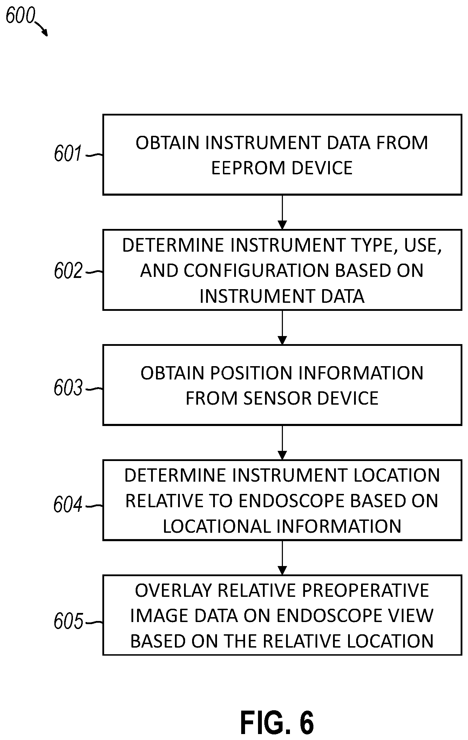

FIG. 6 shows an example of a method (600) through which processor (52) may provide a combination of image (400), preoperative images (510, 520, 530, 540), and one or more overlays, etc., providing additional information about working element (101) or instrument (410). As shown, processor (52) may first obtain (601) instrument data. In this context, the "instrument" may include working element (101) and/or instrument (410). In some cases, the instrument data may be obtained (601) automatically from working element (101) and/or instrument (410) itself. For instance, working element (101) and/or instrument (410) may include an internal storage device (e.g., an EEPROM, etc.), a barcode, or some other feature from which the instrument data may be automatically obtained (601). In addition, or in the alternative, the operator may input the instrument data into processor (52), such as via operating controls (54), etc. Alternatively, the instrument data may be obtained (601) in any other suitable fashion.

Once the instrument data is obtained (601), processor (52) may determine or identify (602) various factors about the instrument use, such as, for example, the instrument type, one or more kinds of instrument use cases (e.g., the kind or kinds of procedures in which the working element (101) and/or instrument (410) is configured to be used), structural characteristics of working element (101) or instrument (410) (e.g., structural characteristics of working element (101) or instrument (410), such as length, bend angle, diameter, etc.), and/or other preoperative information associated with working element (101) or instrument (410).

Once instruments (100, 410) have been inserted into the patient (P), processor (52) may obtain (603) position information from one or more position sensors to determine the real-time position of working element (101) or instrument (410) in the patient (P). As noted above, instrument (100) includes a navigation sensor assembly (110) that may be used to generate such position information. It should be understood that working element (101) or instrument (410) may include a similar kind of position sensor to generate signals indicating the real-time position of working element (101) or instrument (410) in the patient (P).

Based on the obtained (603) position information indicating the real-time position of working element (101) or instrument (410) within the patient (P), processor (52) may determine (604) the location of the instrument relative to instrument (100) (or relative to some other kind of endoscope or other image capturing instrument). As noted above, processor (52) may already "know" the location of instrument (100) within the patient (P) based on signals from navigation sensor assembly (110). Processor (52) may thus correlate the real-time position of working element (101) or instrument (410) within the patient (P) with the real-time position of instrument (100) within the patient (P).

Once processor (52) has determined (602) the instrument information (e.g., type, configuration, use case, etc.) and determined (604) the location of the instrument relative to instrument (100) (or relative to some other kind of endoscope or other image capturing instrument), processor (52) may generate (605) an overlay. This overlay may include a graphical representation of working element (101) or instrument (410), textual information, numerical information, other graphical information (e.g., arrows, etc.). As noted above, the overlay may be positioned on the endoscopic image (400), on one or more preoperative images (510, 520, 530, 540), and/or elsewhere on display screen (56). It should also be understood that more than one overlay may be generated (605). For instance, a graphical representation of working element (101) or instrument (410), and one or more other graphical representations (e.g., an arrow indicating a direction of insertion, etc.), may be overlaid on the endoscopic image (400) and/or on one or more preoperative images (510, 520, 530, 540) on display screen (56); while alphanumeric data (e.g., representing type of instrument, depth of insertion, etc.) may be overlaid on display screen (56) separately from images (400, 520, 530, 540). In addition, or in the alternative, alphanumeric data (e.g., representing type of instrument, depth of insertion, etc.) may be overlaid on the endoscopic image (400) and/or on one or more preoperative images (510, 520, 530, 540) on display screen (56).

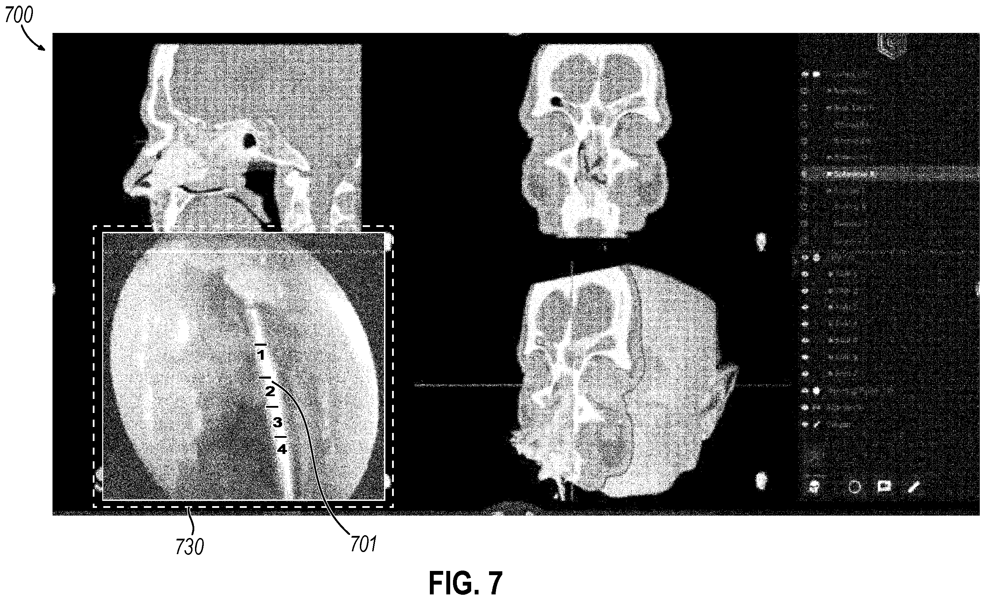

FIG. 7 shows an example of an updated user interface (700), representing an updated version of interface shown in FIG. 5 (500), in which the preoperative image (530) is replaced with an endoscope view (730), similar to the image (400) shown in FIG. 4. Endoscope view (730) includes an actual image (732) of an instrument (e.g., similar to instrument (410)) inserted int the patient (P), adjacent to anatomical structures of the patient (P). However, unlike the basic image of FIG. 4, endoscope view (730) of FIG. 7 also includes an overlay (734) atop the actual image (732) of the instrument within endoscope view (730). In this example, overlay (734) includes indicia showing the depth of insertion of the instrument in the patient (P). Overlay (734) may take various other suitable forms and may provide various other kinds of information, such as the distance of the instrument to a target location within the patient (P), a direction of movement of the instrument within the patient (P), and/or other kinds of information as will be apparent to those skilled in the art in view of the teachings herein. While the additional information is shown in the form of an overlay (734) within endoscope view (730) in this example, at least some additional may be provided in the form of an overlay (734) within any of the preoperative images shown in FIG. 7 and/or in a side region (750) of user interface (700) that is separate from endoscopic view (730) and the preoperative images.

In some variations, the configuration of the instrument itself may change during use of the instrument. For instance, the instrument may have a steerable distal section such that the distal section may have a bend angle that varies during the medical procedure. Similarly, an instrument may have a rotatable shaft or other feature that rotates during the medical procedure. In scenarios where the configuration of the instrument itself changes during use of the instrument in a medical procedure, an overlay (734) may visually indicate those changes in real time. For instance, an overlay (734) may indicate a changing bend angle (or angle of rotation, etc.) in real time with an alphanumeric representation, a graphical representation, or some other visual indication.

Thus, as disclosed herein, in some implementations, an analysis system or method for a medical procedure at an operative site in a volume located inside a patient body may involve the following non-limiting steps. Firstly, a medical procedure type may be selected or determined (e.g., based on preoperative data or user input). Once the procedure has begun, the system may detect, using an image (e.g., an endoscope image such as shown in FIG. 4) of the operative site, at least one instrument present therein, such as instrument (410) shown in FIG. 4. The system may then detect (e.g., via image analysis of the captured image of the operative site, preoperative data, navigational tracking data, etc.), at least one portion of an organ or patient tissue in the vicinity of the detected instrument.

In a further implementation, the system may determine a position of the detected instrument relative to the detected anatomical structure in the patient (e.g., via image analysis of the captured image of the operative site, preoperative data, navigational tracking data, etc.), so that the detected instrument, the endoscope, the detected tissue, and their positions relative to one another are determined. The position of the instrument relative to the endoscope and/or tissue may be used to generate one or more medical procedure parameters defining a medical procedure step. In some implementations, the medical procedure step may be a known step associated with a medical procedure plan. In an alternative implementation, the system may identify or determine the step based on a factor know to the system, such as, for example, the obtained (601) instrument data; the determined (602) instrument type, use case, and configuration; the determined (604) instrument location relative to endoscope; etc.

Once a medical procedure step is identified, the system may, in some implementations, select (e.g., from a database) a conceptual medical procedure step that corresponds to the detected medical procedure step (e.g., the step identified based on information housed in the device on a storage platform, such as an EEPROM device). The system may then compare one or more factors that were identified or determined, as discussed herein, with one or more factors that define the selected conceptual medical procedure step. Based on this comparison, the system may determine what, if any, differences exist between these parameters. If any differences are detected, the system may take various actions, such as, for example, determining that the difference is negligible (thus proceeding with the medical procedure plan); providing a notification to the user of the differences; providing a notification to the user indicating the specific factors causing the difference; requesting user feedback; searching a database for historical medical procedure information that may more closely match the one or more factors; providing information associated with the quality of the detected medical procedure step; etc.

Accordingly, in some implementations, the system may compare the factors of the detected medical procedure step with the factors of a previously performed step or conceptual step. It should be understood that the historical medical procedure information may be information that originated at the medical procedure navigation system as well as historical medical procedure information that originated at other medical procedure navigation systems. Stated differently, in some implementations, the system may access a shared or global database that contains medical procedures performed by other users and/or other medical procedure navigation systems.

Once the system determines the most accurate medical procedure step, it may then display (e.g., on a display screen or wearable device) an overlay on an image (e.g., the image captured by an endoscope) showing a current position of the detected instrument and indicating an optimal position of the instrument. In a further implementation, the system may also display a determined depth of the instrument (e.g., as shown in FIG. 7 by overlay (734)), a bend angle of the instrument, and/or a user-defined usage parameter. In a further implementation, the system may automatically update/modify (e.g., in real time) the overlay image based on the current position of the instrument and/or the most accurate next medical procedure step (e.g., conceptual or historical).

### IV. Example of Combining CT Image View with Endoscopic Image View

As discussed herein, providing supplemental information to a user (e.g., surgeon) during medical procedure navigation may be beneficial for training purposes and/or for improving patient outcomes. This may be especially true when performing procedures within a nasal cavity and/or when performing minimally invasive surgery because only small portions of the instrumentation being used may be visible (e.g., via an endoscope) during the medical procedure. However, further improvements may be possible, such as, for example, showing a high accuracy "virtual" or projected image even when the instrument is not in the vicinity (e.g., field of view) of the endoscope or is otherwise obscured by anatomical structures and/or fluid (e.g., blood). For instance, as shown in FIG. 4, a distal portion (414) of instrument (410) is obscured by a middle turbinate (MT) in a nasal cavity of the patient (P), such that the operator is unable to visualize distal portion (414) distal portion in a conventional endoscopic image (400). However, a proximal portion (412) of instrument (410) is visible in endoscopic image (400). The broken line rendering of distal portion (414) in FIG. 4 is only intended to indicate that distal portion (414) is obscured by the middle turbinate (MT), such that not even a representation of distal portion (414) would be visible in a conventional endoscopic image (400). Only proximal portion (412) would be visible in a conventional endoscopic image (400).

It may therefore be desirable to provide a modified version of endoscopic image (400), to effectively indicate visually to the operator the position of distal portion (414) in relation to surrounding anatomical structures. To the extent that such positional information of distal portion (414) may be conveyed via a set of images (510, 520, 530, 540) such as those shown in user interface (500), or via a set of images such as those shown in user interface (700), it may be further desirable to visually to the operator the position of distal portion (414) in relation to surrounding anatomical structures within a single image. To that end, it may be desirable to effectively combine an endoscopic image (400) (depicting the portion of instrument (410) visible within the field of view of the endoscope (e.g., instrument (100)) with a preoperative image and a real-time position indictor in the preoperative image (to show the otherwise obscured distal portion (414) of instrument (410)). Such a single composite image may more effectively convey the real-time position of distal portion (414) than could be conveyed via a set of images such as those shown in user interfaces (500, 700) described above.

The following describes examples of how a single composite image may be generated to effectively convey the real-time position of a portion of an instrument that is obscured by blood, anatomical structures, etc. while simultaneously displaying the portions of the same instrument that is visible within the field of view of an endoscope. It should be understood that the composite images described below may also include one or more overlay features similar to the overlays described above in connection with FIGS. 5-7. It should therefore be understood that the overlays described above in connection with FIGS. 5-7 and the composite images described below are not mutually exclusive of each other. Various suitable ways in which the overlays described above in connection with FIGS. 5-7 may be incorporated into the composite images described below will be apparent to those skilled in the art in view of the teachings herein.

As noted above, an endoscopic view such as the one depicted in image (400) may be captured by an endoscope or other viewing instrument like instrument (100) of FIGS. 2-3. As also noted above, instrument (100) includes navigation sensor assembly (110) that generates signals indicating the real-time position of distal end (120) within patient (P). Any other kind of endoscope or other viewing instrument may similarly include one or more position sensors like navigation sensor assembly (110). Data from navigation sensor assembly (110) may be indicative of the real-time position of distal end (120) within three-dimensional space, the viewing angle provided by camera (161), the roll angle of shaft assembly (108), etc.

In addition to an endoscope (e.g., instrument (100)) having a position sensor (e.g., navigation sensor assembly (110)), an instrument (e.g., working element (101) or instrument (410)) that is used with the endoscope may also have a position sensor. Such a position sensor may thus be used to determine the real-time position of the instrument in three-dimensional space. In some scenarios, the instrument has known characteristics such as length, width, height, geometry, angle, diameter, etc., that may be used in combination with data from a position sensor of the endoscope to extrapolate the real-time position of the instrument in three-dimensional space. For instance, if a position sensor of the endoscope is used to determine the real-time position of the endoscope in three-dimensional space, and one or more features of the instrument are used to determine (e.g., optically, electromagnetically, etc.) the position of the instrument relative to the endoscope, then the real-time position of the instrument in three-dimensional space may be determined. In addition, certain structural characteristics of the instrument may be predetermined and utilized in determining the real-time position of the instrument in three-dimensional space.

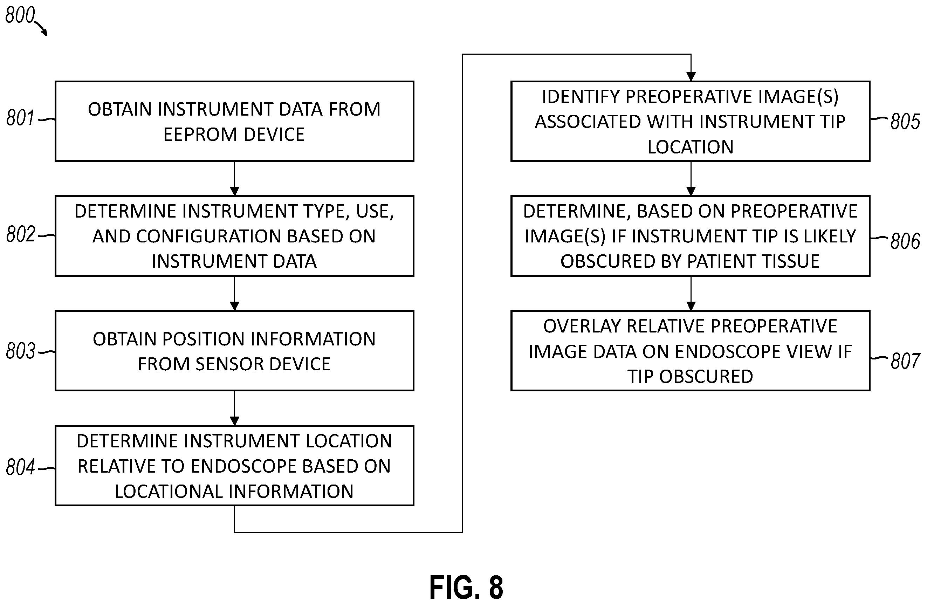

FIG. 8 shows an example of a method (800) where processor (52) may obtain (801) instrument data. In this context, the "instrument" may include working element (101) and/or instrument (410). In some cases, the instrument data may be obtained (601) automatically from working element (101) and/or instrument (410) itself. For instance, working element (101) and/or instrument (410) may include an internal storage device (e.g., an EEPROM, etc.), a barcode, or some other feature from which the instrument data may be automatically obtained (801). In addition, or in the alternative, the operator may input the instrument data into processor (52), such as via operating controls (54), etc. Alternatively, the instrument data may be obtained (801) in any other suitable fashion.

Once the instrument data is obtained (801), processor (52) may determine or identify (802) various factors about the instrument use, such as, for example, the instrument type, one or more kinds of instrument use cases (e.g., the kind or kinds of procedures in which the working element (101) and/or instrument (410) is configured to be used), structural characteristics of working element (101) or instrument (410) (e.g., structural characteristics of working element (101) or instrument (410), such as length, bend angle, diameter, etc.), and/or other preoperative information associated with working element (101) or instrument (410).

Once instruments (100, 410) have been inserted into the patient (P), processor (52) may obtain (803) position information from one or more position sensors to determine the real-time position of working element (101) or instrument (410) in the patient (P). As noted above, instrument (100) includes a navigation sensor assembly (110) that may be used to generate such position information. It should be understood that working element (101) or instrument (410) may include a similar kind of position sensor to generate signals indicating the real-time position of working element (101) or instrument (410) in the patient (P).

Based on the obtained (803) position information indicating the real-time position of working element (101) or instrument (410) within the patient (P), processor (52) may determine (804) the location of the instrument relative to instrument (100) (or relative to some other kind of endoscope or other image capturing instrument). As noted above, processor (52) may already "know" the location of instrument (100) within the patient (P) based on signals from navigation sensor assembly (110). Processor (52) may thus correlate the real-time position of working element (101) or instrument (410) within the patient (P) with the real-time position of instrument (100) within the patient (P).

Processor (52) may further identify (805) one or more preoperative images associated with the real-time position of the distal end of working element (101) or instrument (410); and based on the real-time position of instrument (100) (which will correspond to the position of the field of view of camera (161)). In other words, with the real-time position of the patient (P) having been registered with processor (52) to thereby register the patient (P) with the preoperative images, and with the real-time positions of instrument (100) and either working element (101) or instrument (410) being tracked by processor (52), processor (52) may determine which preoperative images depict the anatomical position in patient (P) where instrument (100) and either working element (101) or instrument (410) are currently located. Moreover, as discussed herein, processor (52) may determine the location of each device relative to the other. Thus, by utilizing a high precision tracking system in combination with known instrumentation processor (52) may determine the real-time locations of the instruments (e.g., instrument (100) and either working element (101) or instrument (410)) inside the body of the patient (P). Based on the location information, processor (52) can then correlate the location of the instruments, and thus the endoscopic view from camera (161), relative to one or more preoperative images.

Once the appropriate preoperative images are identified, processor (52) may determine (806) if a portion of working element (101) or instrument (410) is likely to be obscured by an anatomical structure of the patient (P). This determination (806) may be achieved in a variety of ways. For example, in some versions, processor (52) and/or the operator may rely on the endoscopic view from camera (161) to determine what, if any, portion of working element (101) or instrument (410) is obscured and/or outside the endoscopic view from camera (161). Using the high precision tracking, combined the known factors, processor (52) may construct a projected and/or superimposed view of the location of the obscured or otherwise unseen portion of working element (101) or instrument (410) with refence to a preoperative image (e.g., CT image).

Once processor (52) has determined (802) the instrument information (e.g., type, configuration, use case, etc.) and determined (804) the location of the instrument relative to the endoscope, a graphical representation of the instrument (e.g., its location, size, direction, shape, etc.) may be overlayed (805) with the preoperative image data onto the endoscopic view. In other words, in a region of the endoscopic view where a portion of the inserted instrument is obscured (e.g., by an anatomical structure or by blood, etc.), processor (52) may overlay (805) a corresponding portion of a preoperative image, with a graphical representation of the obscured portion of the inserted instrument in the preoperative image. Processor (52) may thus provide a composite image formed by a combination of the endoscopic view, a portion of a preoperative image, and a graphical representation of an obscured portion of an inserted instrument (e.g., working element (101) or instrument (410)). The portion of the inserted instrument that is not obscured may still be shown in the corresponding portion of the endoscopic view in the composite image. By providing these views within a single composite image, display screen (56) may provide less visual clutter to the operator than might otherwise be provided through the presentation of several separate images simultaneously.

In some variations, the operator may manually designate a region (e.g., via operating controls (54)) within an endoscopic view where processor (52) should overlay a corresponding portion of a preoperative image. Such manual designations may be provided even in scenarios where a portion of an inserted instrument is not being obscured. For instance, an initial endoscopic image may provide a view of a middle turbinate (MT) and surrounding anatomical structures in a nasal cavity, without any preoperative image overlaid on the endoscopic image. The user may select a region within the endoscopic image, such as a portion of the middle turbinate (MT); and processor (52) may then overlay a corresponding preoperative image of that region of the patient's anatomy onto that portion of the endoscopic image.

In some cases (e.g., where the corresponding preoperative image is at a plane that is deeper into the patient anatomy along line of sight), the overlaid preoperative image may allow the operator to effectively "see through" the middle turbinate (MT) or other anatomical structure. This ability to effectively "see through" anatomical structures in a composite image formed by a combination of a real-time endoscopic view and a preoperative image may be provided regardless of whether the composite image is formed via the method (800) of FIG. and/or via an operator's manual selection of a region to "see through." This "see through" capability may also be provided in scenarios where instrumentation or other anatomical structures are obscured in an endoscopic view by blood, other fluids, debris, etc. Similarly, this "see through" capability may also be provided in scenarios where instrumentation or other anatomical structures are otherwise outside the field of view of the endoscope (e.g., instrument (100) or another instrument with a camera).

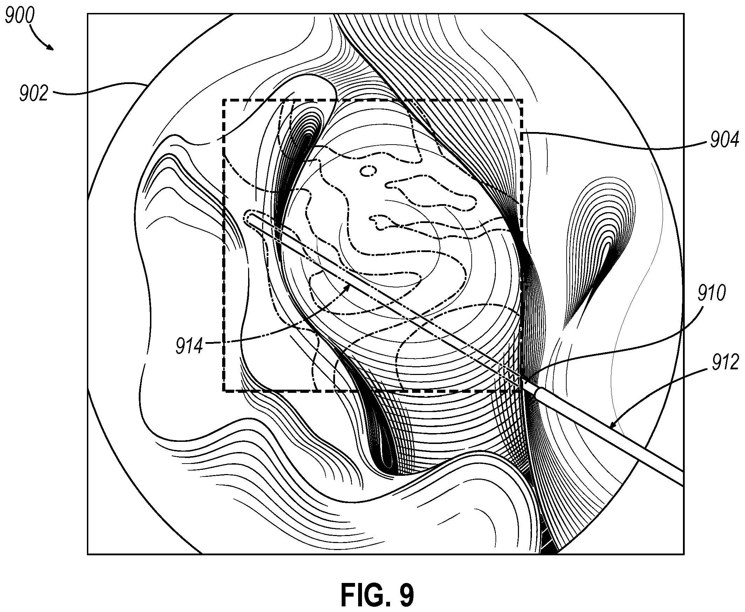

FIG. 9 shows an example of a composite image (900) that may be formed in accordance with the above teachings. Composite image (900) may be rendered on display screen (56) during a medical procedure where an instrument (910) is used in combination with instrument (100) (or in combination with some other endoscope or other camera-equipped instrument). Composite image (900) of this example includes an endoscopic view (902), a semi-transparent overlay (904), and a virtual projection (914). Endoscopic view (902) may include an image captured by camera (161) of instrument (100), by some other kind of endoscope, or by some other kind of instrument with a camera. As can be seen in FIG. 9, a proximal portion (912) of an instrument (910) is visible within endoscopic view (902). Instrument (910) may comprise any of the various kinds of instruments referred to herein; or working element (101), etc.

A distal portion of instrument (910) would be obscured by an anatomical structure in endoscopic view (902), similar to how distal portion (414) is obscured by the middle turbinate (MT) in FIG. 4 as described above. However, processor (52) is able to determine the real-time position of instrument (910) within the patient (P) and relative to the endoscope, which portion of instrument (910) is obscured in endoscopic view (902), which preoperative image region is associated with the anatomy obstructing the obstructed portion of instrument (910), and where that preoperative image region corresponds to the real-time endoscopic view (902). Processor (52) is thus able to select and position that preoperative image region in the form of semi-transparent overlay (904). As shown, in the present example, semi-transparent overlay (904) is generally limited to the region within endoscopic view (902) where the distal portion of instrument (910) is obscured. Thus, semi-transparent overlay (904) does not extend substantially beyond the region within endoscopic view (902) where the distal portion of instrument (910) is obscured. With overlay (904) being semi-transparent, the operator may simultaneously observe endoscopic view (902) and a preoperative image view within the overlay (904) region of composite image (900).

Since processor (52) is also configured to track the real-time position of instrument (910) within the patient (P), processor (52) is operable to generate virtual projection (914) to visually indicate where the otherwise obscured distal portion of instrument (910) is located. In this example, virtual projection (914) is provided only within semi-transparent overlay (904) formed by the corresponding preoperative image region. In some versions where semi-transparent overlay (904) does not span across the entirety of the obscured portion of instrument (910), virtual projection (914) may overlay a portion of endoscopic view (902) where semi-transparent overlay (904) does not extend. In other words, virtual projection (914) may still be used to visually indicate where an obscured portion of instrument (910) is located within endoscopic view (902), even if semi-transparent overlay (904) does not extend into that region of endoscopic view (902). Moreover, processor (52) may be configured to enable the operator to select (e.g., via operating controls (54)) the extent to which semi-transparent overlay (904) should be provided over endoscopic view (902). In some cases, the operator may wish to omit semi-transparent overlay (904) from all or part of a region of endoscopic view (902) in which a distal portion of instrument (910) is obscured, such that the operator may simply rely on virtual projection (914) in endoscopic view (902) to determine where some or all of the obscured portion of instrument (910) is located in relation to anatomical structures.

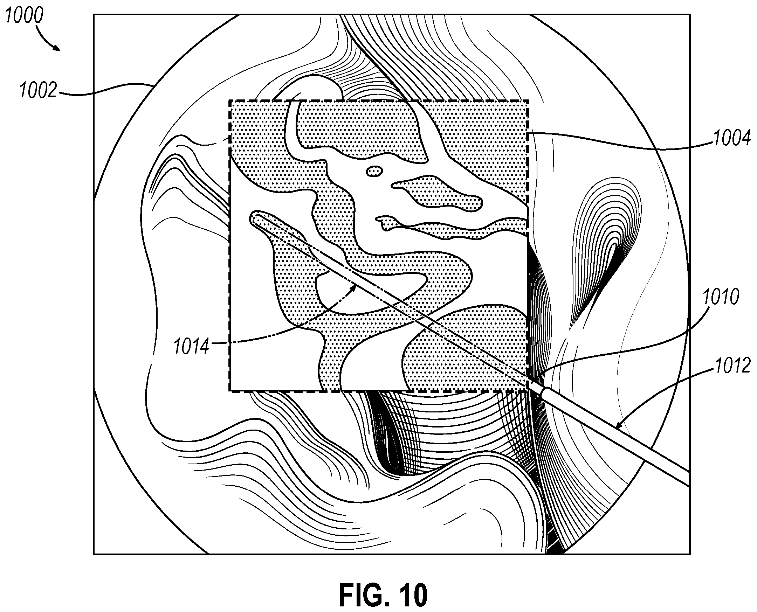

FIG. 10 shows another example of a composite image (1000) that may be formed in accordance with the above teachings. Composite image (1000) may be rendered on display screen (56) during a medical procedure where an instrument (1010) is used in combination with instrument (100) (or in combination with some other endoscope or other camera-equipped instrument). Composite image (1000) of this example includes an endoscopic view (1002), an opaque overlay (1004), and a virtual projection (1014). Endoscopic view (1002) may include an image captured by camera (161) of instrument (100), by some other kind of endoscope, or by some other kind of instrument with a camera. As can be seen in FIG. 10, a proximal portion (1012) of an instrument (1010) is visible within endoscopic view (1002). Instrument (1010) may comprise any of the various kinds of instruments referred to herein; or working element (101), etc.

A distal portion of instrument (1010) would be obscured by an anatomical structure in endoscopic view (1002), similar to how distal portion (414) is obscured by the middle turbinate (MT) in FIG. 4 as described above. However, processor (52) is able to determine the real-time position of instrument (1010) within the patient (P) and relative to the endoscope, which portion of instrument (1010) is obscured in endoscopic view (1002), which preoperative image region is associated with the anatomy obstructing the obstructed portion of instrument (1010), and where that preoperative image region corresponds to the real-time endoscopic view (1002). Processor (52) is thus able to select and position that preoperative image region in the form of opaque overlay (1004). As shown, in the present example, opaque overlay (1004) is generally limited to the region within endoscopic view (1002) where the distal portion of instrument (1010) is obscured. Thus, opaque overlay (1004) does not extend substantially beyond the region within endoscopic view (1002) where the distal portion of instrument (1010) is obscured. With overlay (1004) being opaque, the operator may only view a preoperative image view within the overlay (1004) region of composite image (1000).

Since processor (52) is also configured to track the real-time position of instrument (1010) within the patient (P), processor (52) is operable to generate virtual projection (1014) to visually indicate where the otherwise obscured distal portion of instrument (1010) is located. In this example, virtual projection (1014) is provided only within opaque overlay (1004) formed by the corresponding preoperative image region. In some versions where semi-transparent overlay (1004) does not span across the entirety of the obscured portion of instrument (1010), virtual projection (1014) may overlay a portion of endoscopic view (1002) where opaque overlay (1004) does not extend. In other words, virtual projection (1014) may still be used to visually indicate where an obscured portion of instrument (1010) is located within endoscopic view (1002), even if opaque overlay (1004) does not extend into that region of endoscopic view (1002). Moreover, processor (52) may be configured to enable the operator to select (e.g., via operating controls (54)) the extent to which opaque overlay (1004) should be provided over endoscopic view (1002). In some cases, the operator may wish to omit opaque overlay (1004) from all or part of a region of endoscopic view (1002) in which a distal portion of instrument (1010) is obscured, such that the operator may simply rely on virtual projection (1014) in endoscopic view (1002) to determine where some or all of the obscured portion of instrument (1010) is located in relation to anatomical structures.

As described above, a composite image (900) provides a semi-transparent overlay (904) of a preoperative image region while composite image (1000) provides an opaque overlay (1004) of a preoperative image region. Some variations may allow the operator to select a degree of transparency for a preoperative image overlay (904, 1004) on an endoscopic view (902, 1002). For instance, some versions of processor (52) may allow the operator to select a degree of transparency via operating controls (54), such as through manipulation of a slider, knob, or other control feature, in a graphical user interface or otherwise.

It should also be understood that, as the operator moves instrument (910, 1010) within the patient, the position of overlay (904, 1004) and virtual projection (914, 1014) may correspondingly move in real time to facilitate visual tracking of the movement of instrument (910, 1010) relative to adjacent anatomical structures.

As yet another variation, a composite image (900, 1000) may include one or more additional overlays, such as overlay (734) described above, in addition to including overlay (904, 1004) and virtual projection (914, 1014). In scenarios where more than one instrument (910, 1010) is disposed in the patient simultaneously, and processor (52) is configured to track the real-time position of the two or more instruments (910, 1010) in the patient, each such instrument may have its own corresponding overlay (904, 1004) and virtual projection (914, 1014) within the same single composite image (900, 1000).

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Some versions of the examples described herein may be implemented using a processor, which may be part of a computer system and communicate with a number of peripheral devices via bus subsystem. Versions of the examples described herein that are implemented using a computer system may be implemented using a general-purpose computer that is programmed to perform the methods described herein. Alternatively, versions of the examples described herein that are implemented using a computer system may be implemented using a specific-purpose computer that is constructed with hardware arranged to perform the methods described herein. Versions of the examples described herein may also be implemented using a combination of at least one general-purpose computer and at least one specific-purpose computer.

In versions implemented using a computer system, each processor may include a central processing unit (CPU) of a computer system, a microprocessor, an application-specific integrated circuit (ASIC), other kinds of hardware components, and combinations thereof. A computer system may include more than one type of processor. The peripheral devices of a computer system may include a storage subsystem including, for example, memory devices and a file storage subsystem, user interface input devices, user interface output devices, and a network interface subsystem. The input and output devices may allow user interaction with the computer system. The network interface subsystem may provide an interface to outside networks, including an interface to corresponding interface devices in other computer systems. User interface input devices may include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system.

In versions implemented using a computer system, a storage subsystem may store programming and data constructs that provide the functionality of some or all of the modules and methods described herein. These software modules may be generally executed by the processor of the computer system alone or in combination with other processors. Memory used in the storage subsystem may include a number of memories including a main random-access memory (RAM) for storage of instructions and data during program execution and a read only memory (ROM) in which fixed instructions are stored. A file storage subsystem may provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem in the storage subsystem, or in other machines accessible by the processor.

In versions implemented using a computer system, the computer system itself may be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the example of the computer system described herein is intended only as a specific example for purposes of illustrating the technology disclosed. Many other configurations of a computer system are possible having more or fewer components than the computer system described herein.

Having shown and described various embodiments of the present invention, further adaptations of the methods and systems described herein may be accomplished by appropriate modifications by one of ordinary skill in the art without departing from the scope of the present invention as defined by the appended claims. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A system comprising:
(a) a medical procedure instrument (101, 410, 910, 1010) having a first position sensor assembly (110);
(b) an imaging instrument (100, 161) having a second position sensor assembly; and
(c) a processor (52), the processor being configured to:
(i) determine a real-time position of the medical procedure instrument based on a signal from the first position sensor assembly,
(ii) determine a real-time position of the imaging instrument based on a signal from the second position sensor assembly,
(iii) display a real-time image from the imaging instrument, the real-time image providing a field of view,
(iv) determine that a portion of the medical procedure instrument is obscured within the field of view,
(v) select a preoperative image region corresponding to the obscured portion of the medical procedure instrument, and
(vi) overlay the selected preoperative image region onto the real-time image from the imaging instrument, at a location corresponding to a region of the field of view in which the portion of the medical instrument is obscured.

2. The system of claim 1, the medical procedure instrument being selected from the group consisting of a dilation catheter, an ablation instrument, a curette, a microdebrider or other shaver, a probe, an irrigation instrument, and a suction instrument.

3. The system of claim 1 or claim 2, the imaging instrument comprising an endoscope.

4. The system of any of claims 1 to 3, the imaging instrument having a working channel, the medical procedure instrument being disposed within the working channel.

5. The system of any of claims 1 to 4, the processor being further configured to render a virtual representation (914) of the obscured portion of the medical procedure instrument at a location corresponding to a region of the field of view in which the portion of the medical instrument is obscured, optionally the processor being further configured to render the virtual representation within the overlaid preoperative image region.

6. The system of any of claims 1 to 5, the processor being further configured to:
(i) receive a user selection indicating a transparency level, and
(ii) apply a transparency level to the overlay (904, 1004) based on the user selection.

7. The system of claim 1, the processor being further configured to provide a secondary overlay (734) on the real-time image from the imaging instrument, the secondary overlay representing a preoperative characteristic of the medical procedure instrument or a real-time operational characteristic of the medical procedure instrument.

8. The system of any preceding claim, the processor being further configured to:
(a) determine an insertion depth of the medical procedure instrument within patient anatomy; and
(b) display, on the display device, a visual representation (734) of the determined insertion depth.

9. The system of any preceding claim, the processor being further configured to:
(a) determine a bend angle of the instrument; and
(b) display, on the display device, a visual representation of the determined bend angle.

10. The system of any preceding claim, the processor being further configured to:
(a) receive, via user input, at least one user criteria; and
(b) display, on the display device, a visual representation of the at least one user criteria.

11. The system of any preceding claim, the medical procedure instrument and the imaging instrument being housed together in a single instrument (100).

## Patentansprüche

1. System, umfassend:
(a) ein medizinisches Eingriffsinstrument (101, 410, 910, 1010) mit einer ersten Positionssensoranordnung (110);
(b) ein Bildgebungsinstrument (100, 161) mit einer zweiten Positionssensoranordnung; und
(c) einen Prozessor (52), wobei der Prozessor ausgelegt ist zum:
(i) Bestimmen einer Echtzeitposition des medizinischen Eingriffsinstruments basierend auf einem Signal von der ersten Positionssensoranordnung,
(ii) Bestimmen einer Echtzeitposition des Bildgebungsinstruments basierend auf einem Signal von der zweiten Positionssensoranordnung,
(iii) Anzeigen eines Echtzeitbildes von dem Bildgebungsinstrument, wobei das Echtzeitbild ein Sichtfeld bereitstellt,
(iv) Bestimmen, dass ein Abschnitt des medizinischen Eingriffsinstruments innerhalb des Sichtfeldes verdeckt ist,
(v) Auswählen einer präoperativen Bildregion, die dem verdeckten Abschnitt des medizinischen Eingriffsinstruments entspricht, und
(vi) Überlagern der ausgewählten präoperativen Bildregion von dem Bildgebungsinstrument auf das Echtzeitbild an einem Ort, der einer Region des Sichtfeldes entspricht, worin der Abschnitt des medizinischen Instruments verdeckt ist.

2. System nach Anspruch 1, wobei das medizinische Eingriffsinstrument ausgewählt ist aus der Gruppe bestehend aus einem Dilatationskatheter, einem Ablationsinstrument, einer Kürette, einem Mikrodebrider oder anderem Shaver, einer Sonde, einem Irrigationsinstrument und einem Absauginstrument.

3. System nach Anspruch 1 oder Anspruch 2, wobei das Bildgebungsinstrument ein Endoskop umfasst.

4. System nach einem der Ansprüche 1 bis 3, wobei das Bildgebungsinstrument einen Arbeitskanal aufweist, wobei das medizinische Eingriffsinstrument innerhalb des Arbeitskanals angeordnet ist.

5. System nach einem der Ansprüche 1 bis 4, wobei der Prozessor ferner dazu ausgelegt ist, eine virtuelle Darstellung (914) des verdeckten Abschnitts des medizinischen Eingriffsinstruments an einem Ort zu rendern, der einer Region des Sichtfeldes entspricht, worin der Abschnitt des medizinischen Instruments verdeckt ist, wobei gegebenenfalls der Prozessor ferner dazu ausgelegt ist, die virtuelle Darstellung innerhalb des überlagerten präoperativen Bildbereichs zu rendern.

6. System nach einem der Ansprüche 1 bis 5, wobei das System weiterhin ausgelegt ist zum:
(i) Empfangen einer Benutzerauswahl, die ein Transparenznivau angibt, und
(ii) Anwenden eines Transparenzniveaus auf die Überlagerung (904, 1004) basierend auf der Benutzerauswahl.

7. System nach Anspruch 1, wobei der Prozessor ferner dazu ausgelegt ist, eine sekundäre Überlagerung (734) auf dem Echtzeitbild von dem Bildgebungsinstrument bereitzustellen, wobei die sekundäre Überlagerung eine präoperative Charakteristik des medizinischen Eingriffsinstruments oder eine Echtzeit-Betriebscharakteristik des medizinischen Eingriffsinstruments repräsentiert.

8. System nach einem der vorhergehenden Ansprüche, wobei der Prozessor ferner ausgelegt zum:
(a) Bestimmen einer Einführtiefe des medizinischen Eingriffsinstruments innerhalb der Patientenanatomie; und
(b) Anzeigen einer visuellen Darstellung (734) der bestimmten Einführtiefe auf der Anzeigevorrichtung.

9. System nach einem der vorhergehenden Ansprüche, wobei der Prozessor ferner ausgelegt zum:
(a) Bestimmen eines Biegewinkels des Instruments; und
(b) Anzeigen einer visuellen Darstellung des bestimmten Biegewinkels auf der Anzeigevorrichtung.

10. System nach einem der vorhergehenden Ansprüche, wobei der Prozessor ferner ausgelegt zum:
(a) Empfangen mindestens eines Benutzerkriteriums über eine Benutzereingabe; und
(b) Anzeigen einer visuellen Darstellung des mindestens einen Benutzerkriteriums auf der Anzeigevorrichtung.

11. System nach einem der vorhergehenden Ansprüche, wobei das medizinische Eingriffsinstrument und das Bildgebungsinstrument zusammen in einem einzigen Instrument (100) untergebracht sind.

## Revendications

1. Système comprenant :
(a) un instrument d'intervention médicale (101, 410, 910, 1010) ayant un premier ensemble de capteurs de position (110) ;
(b) un instrument d'imagerie (100, 161) ayant un second ensemble de capteurs de position ; et
(c) un processeur (52), le processeur étant configuré pour :
(i) déterminer une position en temps réel de l'instrument d'intervention médicale sur la base d'un signal provenant du premier ensemble de capteurs de position,
(ii) déterminer une position en temps réel de l'instrument d'imagerie sur la base d'un signal provenant du second ensemble de capteurs de position,
(iii) afficher une image en temps réel provenant de l'instrument d'imagerie, l'image en temps réel fournissant un champ de vision,
(iv) déterminer qu'une partie de l'instrument d'intervention médicale est masquée dans le champ de vision,
(v) sélectionner une région d'image préopératoire correspondant à la partie masquée de l'instrument d'intervention médicale, et
(vi) superposer la région d'image préopératoire sélectionnée sur l'image en temps réel provenant de l'instrument d'imagerie, à un emplacement correspondant à une région du champ de vision dans laquelle la partie de l'instrument médical est masquée.

2. Système selon la revendication 1, l'instrument d'intervention médicale étant choisi dans le groupe constitué d'un cathéter de dilatation, d'un instrument d'ablation, d'une curette, d'un microdébrideur ou d'un autre rasoir, d'une sonde, d'un instrument d'irrigation et d'un instrument d'aspiration.

3. Système selon la revendication 1 ou la revendication 2, l'instrument d'imagerie comprenant un endoscope.

4. Système selon l'une quelconque des revendications 1 à 3, l'instrument d'imagerie ayant un canal de travail, l'instrument d'intervention médicale étant disposé à l'intérieur du canal de travail.

5. Système selon l'une quelconque des revendications 1 à 4, le processeur étant en outre configuré pour rendre une représentation virtuelle (914) de la partie masquée de l'instrument d'intervention médicale à un emplacement correspondant à une région du champ de vision dans laquelle la partie de l'instrument médical est masquée, éventuellement le processeur étant en outre configuré pour rendre la représentation virtuelle à l'intérieur de la région d'image préopératoire superposée.

6. Système selon l'une quelconque des revendications 1 à 5, le processeur étant en outre configuré pour :
(i) recevoir une sélection de l'utilisateur indiquant un niveau de transparence, et
(ii) appliquer un niveau de transparence à la superposition (904, 1004) sur la base de la sélection de l'utilisateur.

7. Système selon la revendication 1, le processeur étant en outre configuré pour fournir une superposition secondaire (734) sur l'image en temps réel provenant de l'instrument d'imagerie, la superposition secondaire représentant une caractéristique préopératoire de l'instrument d'intervention médicale ou une caractéristique opérationnelle en temps réel de l'instrument d'intervention médicale.

8. Système selon l'une quelconque des revendications précédentes, le processeur étant en outre configuré pour :
(a) déterminer une profondeur d'insertion de l'instrument d'intervention médicale dans l'anatomie du patient ; et
(b) afficher, sur le dispositif d'affichage, une représentation visuelle (734) de la profondeur d'insertion déterminée.

9. Système selon l'une quelconque des revendications précédentes, le processeur étant en outre configuré pour :
(a) déterminer un angle de courbure de l'instrument ; et
(b) afficher, sur le dispositif d'affichage, une représentation visuelle de l'angle de courbure déterminé.

10. Système selon l'une quelconque des revendications précédentes, le processeur étant en outre configuré pour :
(a) recevoir, par saisie utilisateur, au moins un critère utilisateur ; et
(b) afficher, sur le dispositif d'affichage, une représentation visuelle de l'au moins un critère utilisateur.

11. Système selon l'une quelconque des revendications précédentes, l'instrument d'intervention médicale et l'instrument d'imagerie étant logés ensemble dans un seul instrument (100).
